(19) Europäisches Patentamt
European Patent Office
Office européen des brevets

(11) Veröffentlichungsnummer : **0 378 081 B1**

(12) **EUROPÄISCHE PATENTSCHRIFT**

(45) Veröffentlichungstag der Patentschrift :
**31.03.93 Patentblatt 93/13**

(51) Int. Cl.$^5$ : **C07C 47/127,** C07C 45/78, B01D 61/24, B01D 61/44

(21) Anmeldenummer : **90100056.2**

(22) Anmeldetag : **03.01.90**

# (54) Verfahren zur Reinigung wässriger Glyoxallösungen.

(30) Priorität : **09.01.89 DE 3900379**

(43) Veröffentlichungstag der Anmeldung :
**18.07.90 Patentblatt 90/29**

(45) Bekanntmachung des Hinweises auf die Patenterteilung :
**31.03.93 Patentblatt 93/13**

(84) Benannte Vertragsstaaten :
**BE CH DE FR GB IT LI**

(56) Entgegenhaltungen :
**GB-A- 1 130 760**
**US-A- 3 507 764**

(56) Entgegenhaltungen :
**CHEMCIAL ABSTRACTS, Band 94, Nr. 4, 26. Januar 1981, Seite 354, Zusammenfassung Nr. 20979v, Columbus, Ohio, US ; B.M. FELDMAN : "Some problems of the electrodialysis purification of aqueous solutions of glyoxal" & ElektroKhimiya Ionitov Krasnodar 1979, 62-7**
**KIRK-OTHMER: "ENCYCLOPEDIA OF CHEMICAL TECHNOLOGY", 3. Ausgabe, Band 8, 1979, Seite 729, Verlag John Wiley & Sons, New York, US**

(73) Patentinhaber : **BASF Aktiengesellschaft**
**Carl-Bosch-Strasse 38**
**W-6700 Ludwigshafen (DE)**

(72) Erfinder : **Steiniger, Michael, Dr.**
**Wolfskeule 12**
**W-6730 Neustadt (DE)**
Erfinder : **Voss, Hartwig, Dr.**
**Mozartstrasse 57**
**W-6710 Frankenthal (DE)**
Erfinder : **Hupfer, Leopold, Dr.**
**Waltershoehe 3**
**W-6701 Friedelsheim (DE)**

**EP 0 378 081 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein Verfahren zur Reinigung wäßriger Glyoxallösungen mit Hilfe einer Membranzelle.

Glyoxal wird bekanntlich durch Oxidation von Acetaldehyd mit Salpetersäure oder durch katalytische Dehydrierung von Ethylenglykol hergestellt. Bei diesen Verfahren fallen wäßrige Glyoxallösungen an, die mit organischen Säuren, wie Ameisensäure, Essigsäure, Glykolsäure, Glyoxylsäure und Oxalsäure verunreinigt sind und üblicherweise Säurezahlen von 5 bis 200 aufweisen.

Da man an die Reinheit des Glyoxals, das man als Hilfsmittelkomponente in der Textil- oder Papierindustrie verwendet, hohe Anforderungen stellt und z.B. eine Säurezahl von < 1 fordert, hat es nicht an Vorschlägen gefehlt, die rohen wäßrigen Glyoxallösungen zu reinigen.

Nach den Angaben der US-PS 3 270 062 werden rohe wäßrige Glyoxallösungen durch Behandlung mit festen Ionenaustauschern gereinigt. Der Nachteil dieses Verfahrens liegt in der aufwendigen Regenerierung der Ionenaustauscher, die wegen der hohen Säurewerte der Lösungen häufig durchzuführen ist.

Aus der US-PS 3 860 656 und der DE-PS 34 02 733 ist bekannt, daß sich die sauren Verunreinigungen durch Behandlung der rohen wäßrigen Glyoxallösungen mit Lösungen hochmolekularer tertiärer Amine entfernen lassen. Nachteilig ist hierbei der Umstand, daß die organische Aminphase in einem separaten Verfahrensschritt, z.B. mit wäßrigen Lösungen alkalisch wirkender Mittel, regeneriert werden muß.

In der US 3 507 764 wird ein Membranverfahren beschrieben, bei dem man die Abreicherung der organischen Säuren in den rohen Glyoxallösungen durch Elektrodialyse vornimmt. Bei diesem Verfahren wird die wäßrige rohe Glyoxallösung durch Kammern einer Elektrodialysezelle geleitet, die auf der einen Seite durch Kationenaustauschermembranen und auf der anderen Seite durch Anionenaustauschermembranen begrenzt sind. Dieses Verfahren ist insofern unzureichend, als es nur die Reinigung relativ verdünnter Glyoxallösungen erlaubt. So geht z.B. bei Verwendung höherkonzentrierter Lösungen, die z.B. einen Glyoxalgehalt von etwa 40 % aufweisen, etwa 25 % des Glyoxals durch unerwünschte Überführung in die Konzentratkammern verloren. Die Aufkonzentrierung der gereinigten verdünnten Glyoxallösungen in die handelsüblichen Lösungen, die einen Glyoxalgehalt von 40 % aufweisen, ist mit zusätzlichem Energieaufwand verbunden.

Es bestand somit die Aufgabe, ein Verfahren zu finden, das es gestattet aus den verunreinigten wäßrigen Glyoxallösungen unter Vermeidung der zuvor beschriebenen Nachteile möglichst hochkonzentrierte gereinigte Glyoxallösungen mit einer Säurezahl < 1 zu gewinnen.

Es wurde nun gefunden, daß man wäßrige rohe Glyoxallösungen, die organische Säuren enthalten, mittels einer aus mindestens zwei Kammern bestehenden Membranzelle, in der die Kammern durch Ionenaustauschermembranen voneinander getrennt sind, besonders vorteilhaft reinigen kann, wenn man die Glyoxallösung durch mindestens eine der Kammern der Zelle, die von der (den) benachbarten Kammer(n) durch Anionentauschermembranen getrennt ist (sind), leitet und wobei man durch mindestens eine der benachbarten Kammern die wäßrige Lösung einer Base leitet.

Man kann nach dem neuen Verfahren wäßrige Glyoxallösungen reinigen, die z.B. bei der an sich bekannten Salpetersäure-Oxidation von Acetaldehyd oder Dehydrierung von Glykol erhalten werden, und die üblicherweise Säurezahlen von 5 bis 200 aufweisen. Geeignet sind z.B. wäßrige Glyoxallösungen der genannten Art mit einem Glyoxalgehalt von 20 bis 50, insbesondere 35 bis 45 Gew.-%.

Als Membranzellen verwendet man die an sich bekannten, mit Austauschermembranen und Dichtrahmen ausgerüsteten Apparate, die bis zu 800, zweckmäßig bis zu 500 parallel zueinander angeordnete Kammern enthalten. Vorzugsweise enthalten die Membranzellen 1 bis 400 Kammern. Die Abstände der Membranen zueinander betragen 10 bis 0,4, vorzugsweise 4 bis 0,4 mm.

Als Basen werden wasserlösliche basisch wirkende Stoffe verwendet, zweckmäßigerweise Alkali-, Erdalkali- oder Ammoniumsalze der Kohlensäure, wie die Carbonate, Hydrogencarbonate der Alkali- oder Erdalkalimetalle oder des Ammoniums oder quartäre Ammoniumcarbonate oder -hydrogencarbonate. Die eingesetzten wäßrigen Lösungen der Basen haben je nach Konzentration einen unterschiedlichen pH-Wert. Bevorzugt sind solche wäßrigen Basenlösungen, die einen pH-Wert zwischen 7,5 und 10, insbesondere zwischen 8 und 8,5 aufweisen.

Man führt das Verfahren der Erfindung z.B. so durch, daß man die zu reinigende rohe Glyoxallösung durch die Kammern $K_1$ und die wäßrige Lösung der Base durch die Kammern $K_2$ einer Dialysezelle oder Elektrodialysezelle mit der Sequenz

$$-(-K_2-M_1-K_1-M_1-)_n- \qquad I,$$

in der $M_1$ eine Anionenaustauschermembran und n eine Zahl von 1 bis 800 bedeuten, leitet.

Die Dialysezelle mit der oben bezeichneten Sequenz I kann auch als Elektrodialysezelle betrieben werden. In diesem Falle wird das elektrische Feld auf an sich bekannte Weise mittels der beiden am jeweiligen Ende des Membranstapels angelegten Elektroden erzeugt. Man elektrodialysiert mit Gleichstrom und Stromdichten von 0,1 bis 10 A/dm², vorzugsweise 0,5 bis 3 A/dm². Die dafür notwendige Gleichspannung ist von den Leitfähigkeiten der Lösungen und Membranen sowie von den Membranabständen abhängig. Als Elektrodenspüllösung verwendet man die dafür an sich üblichen Elektrolyten, wie eine 0,5 bis 10 %ige wäßrige Natriumsulfat-oder Natriumacetat-Lösung.

Man kann das Verfahren der Erfindung auch in Elektrodialysezellen mit der charakteristischen Sequenz

$$-(-M_2-K_2-M_1-K_1-M_1-K_3-)_n- \qquad II,$$

durchführen, wobei man die zu reinigende wäßrige Glyoxallösung durch die Kammern $K_1$, die wäßrige Base durch die Kammern $K_2$ und einen im Prinzip beliebigen Elektrolyten durch die Kammern $K_3$ leitet. $M_2$ bedeutet eine bipolare Membran oder eine Kationenaustauschermembran. $M_1$ und n haben die obengenannte Bedeutung.

Auch in diesem Falle wird das elektrische Feld wie oben beschrieben an den Enden des Membranstapels angelegt und die Elektrodialyse wie oben angegeben durchgeführt. Dabei muß das elektrische Feld so angelegt werden, daß die aus $K_1$ zu entfernenden Säureanionen nach $K_3$ elektrisch überführt werden.

Als Elektrolyten, den man durch die Kammern $K_3$ leitet, verwendet man einen wäßrigen Elektrolyten, beispielsweise eine wäßrige Lösung von Salzen, wie Natriumchlorid oder Natriumsulfat oder von Säuren, wie niedere Carbonsäuren. Bevorzugt werden wäßrige Lösungen von Essigsäure oder Acetaten der Alkalimetalle eingesetzt, wie eine 0,5 bis 0,01 normale Essigsäure oder eine 0,5 bis 0,01 normale Natriumacetat-Lösung.

Als Ionenaustauschermembranen kommen an sich bekannte Membranen in Betracht, die z.B. eine Dicke von 0,1 bis 1 mm und einen Porendurchmesser von 1 bis 30 µm oder eine gelartige Struktur aufweisen. Die Anionenaustauschermembranen sind üblicherweise aus einem Matrixpolymer aufgebaut, das chemisch gebundene kationische Gruppen enthält, während die Kationenaustauschermembranen ein Matrixpolymer mit anionischen Gruppen aufweisen. Die bipolaren Membranen weisen auf der einen Oberflächenseite anionische und auf der anderen Oberflächenseite kationische Gruppen auf. Ionenaustauschermembranen der genannten Art sind z.B. die unter den Bezeichnungen SELEMION® (Asahi Glass), NEOSEPTA® (Tokoyama Soda) oder IONAC® (Ionac Chemical Company) im Handel erhältlichen Membranen auf Polystyrolbasis. Die bipolaren Membranen können z.B. durch Zusammenlegen von Kationen- und Anionenaustauschermembranen, durch Verkleben von Kationen- und Anionenaustauschermembranen (s. z.B. DE-OS 35 08 206) oder als "single-film"-Membran, wie z.B. in der US-PS 4 057 481 beschrieben, hergestellt werden.

Das erfindungsgemäße Verfahren, welches sowohl kontinuierlich als auch diskontinuierlich durchgeführt werden kann, wird bei Temperaturen von 0 bis 100°C, vorzugsweise bei 10 bis 45°C und bei Drücken von 1 bis 10 bar, vorzugsweise bei Atmosphärendruck betrieben. Die durch die Membranzellen geführten Lösungen haben eine Strömungsgeschwindigkeit von 0,001 bis 2,0 m/s, vorzugsweise 0,01 bis 0,1 m/s.

Nach dem erfindungsgemäßen Verfahren wird überraschenderweise trotz hoher Glyoxalkonzentration ein hoher Reinigungseffekt bei sehr geringen Glyoxalverlusten erzielt.

Beispiele 1 und 2

Es wurde eine Dialysezelle vom Typ I mit n = 10 verwendet, die als Membranen ($M_1$) im Handel unter der Bezeichnung SELEMION® AMV (Firma Asahi Glass) erhältliche Anionenaustauschermembranen enthielt. Die Dialysezelle war so aufgebaut, daß sich an beiden Enden je eine Kammer $K_2$ befand. Die effektive Fläche pro Membran betrug 0,375 dm²; die effektive Gesamtmembranfläche der Dialysezelle betrug somit 7,5 dm². Der Abstand zwischen der benachbarten Membranen lag bei 3 mm.

Über Vorratsgefäße wurden durch die Kammern $K_1$ die zu reinigende rohe wäßrige Glyoxallösung und durch die Kammern $K_2$ 1000 g einer verdünnten wäßrigen Lösung von Natriumhydrogencarbonat mit einem pH-Wert von 8 bei 25°C im Kreis gepumpt, bis der pH-Wert der Glyoxallösung auf dem gewünschten Wert angestiegen war. Weitere Angaben, wie solche über die Einsatzmengen, die Zusammensetzung der Rohglyoxallösung, die Versuchsbedingungen sowie die Ergebnisse sind der Tabelle zu entnehmen.

Beispiele 3 bis 5

Es wurde eine Dialysezelle verwendet, wie sie in den Beispielen 1 und 2 beschrieben ist, die jedoch an den beiden Enden des Membranstapels mit je einer Platinelektrode der Oberfläche 35 $cm^2$ und mit den dazugehörigen Elektrodenspülkreisen (als Elektrolyt diente eine 5 %ige wäßrige Natriumsulfatlösung) ausgestattet war. Die beiden Elektrolyträume waren von den angrenzenden Kammern $K_2$ durch eine im Handel unter der Bezeichnung NAFION® (Firma Dupont) erhältliche Kationenaustauschermembran getrennt. In dieser Elektrodialysezelle wurden eine rohe Glyoxallösung bei konstanter Klemmspannung und abfallender Stromstärke bei 25°C elektrodialysiert, bis der pH-Wert der Glyoxallösung auf den gewünschten Wert angesteigen war. Durch die Kammern $K_2$ wurde analog den Beispielen 1 und 2 1000 g einer verdünnten wäßrigen Natriumhydrogencarbonatlösung (pH=8) geleitet. Einsatzmengen und Zusammensetzungen der Rohglyoxallösungen, Versuchsbedingungen sowie die Ergebnisse sind der Tabelle zu entnehmen.

Beispiele 6 und 7

Es wurde eine Elektrodialysezelle mit der Sequenz

$$-M_2-K_2-M_1-K_1-M_1-K_3-M_2-$$

verwendet, an deren Enden sich die Elektrodenspülkreise des Katholyt- bzw. Anolytraumes befanden. Als Membranen $M_1$ wurden die im Handel unter der Bezeichnung SELEMION® AMV (Firma Asahi Glass) erhältlichen Anionenaustauschermembranen und als Membranen $M_2$ bipolaren Membranen verwendet. Die bipolaren Membranen wurden durch Zusammenlegen einer SELEMION® AMV Anionenaustauschermembran und einer SELEMION® CMV Kationenaustauschermembran erhalten. Die effektive Fläche der Membranen betrug 3,14 $cm^2$. Der Abstand zwischen den Membranen lag bei 1 cm.

Die rohe Glyoxallösung wurde über ein Vorratsgefäß bei 34°C durch die Kammer $K_1$ im Kreis gepumpt. Durch die Kammer $K_2$ wurden 80 g einer wäßrigen Natriumhydrogencarbonatlösung (C=0,5 eq/kg) geleitet. Während der Elektrodialyse wurde die Konzentration an Hydrogencarbonat-Anionen durch eine durch den pH-Wert gesteuerte Dosierung von Kohlendioxid aufrecht erhalten. Dabei wurde ein pH-Wert von 8 bis 8,2 eingehalten. In einem dritten Kreislauf wurden 80 g einer verdünnten Essigsäure (C=0,1 eq/kg) durch die Kammer $K_3$ gepumpt. Für den Elektrodenspülkreis wurden als Elektrolyt 150 g einer verdünnten wäßrigen Natriumacetatlösung V:(C=0,1 eq/kg) verwendet.

Die Elektrodialyse wurde bei konstanter Klemmenspannung und abfallender Stromstärke (vgl. Tabelle) durchgeführt, bis der pH-Wert der Glyoxallösung auf den gewünschten Wert angestiegen war. Einsatzmengen und Zusammensetzung der Rohglyoxallösungen, Versuchsbedingungen sowie die Ergebnisse sind der Tabelle zu entnehmen.

Beispiel 8

Es wurde die in den Beispielen 6 und 7 beschriebene Elektrodialysezelle eingesetzt, in der jedoch die bipolaren Membranen $M_2$ durch die im Handel unter der Bezeichnung SELEMION® CMV (Firma: Asahi Glass) erhältlichen Kationenaustauschmembranen ersetzt waren.

Über Vorratsgefäße wurden die Rohglyoxallösung bei 30°C durch die Kammer $K_1$, 80 g einer verdünnten wäßrigen Natriumhydrogencarbonat (pH=8) durch die Kammer $K_2$ und 80 g einer verdünnten wäßrigen Natriumacetatlösung (C=0,1 eg/kg) durch die Kammer $K_3$ im Kreis gepumpt. Der Elektrodenspülkreis war mit 150 g einer verdünnten wäßrigen Natriumsulfatlösung (C=0,1 eq/kg) beschickt. Die verwendeten Membranen hatten eine effektive Fläche von 3,14 $m^2$, der Membranabstand betrug 1 cm. Die Elektrodialyse wurde bei konstanter Klemmenspannung und abfallender Stromstärke (vgl. Tabelle) durchgeführt, bis der pH-Wert der Glyoxallösung auf den gewünschten Wert angestiegen war. Einsatzmenge und Zusammensetzung der Rohglyoxallösung, Versuchsbedingungen sowie die Ergebnisse sind der Tabelle zu entnehmen.

Ergebnisse der Beispiele 1 - 8

| | Menge (g) | Glyoxal Konzentration (Gew.%) | pH | SZ[1] | $x^{2)}$ (mS/cm) | Klemmenspannung (V) | Strom (mA) | Dauer (h) | Ladungsmenge (Ah) | Materialausbeute (%) | Säureabreicherung (%) |
|---|---|---|---|---|---|---|---|---|---|---|---|
| Rohglyoxal Einsatz Beispiele 1-4 | 1000 | 40 | 1,3 | 13 | | | | | | | |
| Austrag Beispiel 1 | 1039 | 34,8 | 5,5 | 0,17 | 0,22 | - | - | 13,5 | - | 90 | 99 |
| Austrag Beispiel 2 | 1060 | 37,5 | 3 | 1,7 | 0,20 | - | - | 10,0 | - | 99 | 87 |
| Austrag Beispiel 3 | 1038 | 37,4 | 3 | 1,1 | 0,15 | 50 | 330-230 | 6,5 | 1,6 | 96 | 91 |
| Austrag Beispiel 4 | 1043 | 37,4 | 5,7 | 0,34 | 0,19 | 100 | 640-470 | 5,7 | 3,1 | 97 | 97 |
| Rohglyoxal Einsatz Beispiel 5 | 1000 | 42 | 1,5 | 6,6 | | | | | | | |
| Austrag Beispiel 5 | 1026 | 40 | 5,0 | 0,61 | 0,15 | 50 | 290-190 | 5,5 | 1,1 | 97 | 91 |
| Rohglyoxal Einsatz Beispiel 6 | 60 | 40,5 | 1,45 | 9,3 | 0,68 | | | | | | |

Ergebnisse der Beispiele 1 - 8

| | Menge (g) | Glyoxal Konzentration (Gew.%) | pH | SZ1) | x2) (mS/cm) | Klemmenspannung (V) | Strom (mA) | Dauer (h) | Ladungsmenge (Ah) | Materialausbeute (%) | Säureabreicherung (%) |
|---|---|---|---|---|---|---|---|---|---|---|---|
| Austrag Beispiel 6 | 60 | 39,5 | 5 | 0,59 | 0,23 | 150 | 110-50 | 8 | 0,62 | 97 | 94 |
| Rohglyoxal Einsatz Beispiele 7-8 | 60,5 | 42,2 | 1,55 | 6,25 | 0,78 | | | | | | |
| Austrag Beispiel 7 | 60,8 | 40,7 | 5,1 | 0,67 | 0,15 | 150 | 143-49 | 5,5 | 0,41 | 97 | 89 |
| Austrag Beispiel 8 | 60,7 | 41,2 | 5,1 | 0,74 | 0,14 | 100 | 138-54 | 4,8 | 0,39 | 98 | 88 |

1) SZ: Säurezahl mg KOH/g Substanz
2) Leitfähigkeit

## Patentansprüche

1. Verfahren zur Reinigung wäßriger Glyoxallösungen, die organische Säuren enthalten, mittels einer aus mindestens zwei Kammern bestehenden Membranzelle, in der die Kammern durch Ionenaustauschermembranen voneinander getrennt sind, dadurch gekennzeichnet, daß man die Glyoxallösung durch mindestens eine der Kammern der Zelle, die von der (den) benachbarten Kammer(n) durch Anionenaustauschermembranen getrennt ist (sind), leitet und wobei man durch mindestens eine der benachbarten Kammern die wäßrige Lösung einer Base leitet.

2. Verfahren nach Anspruch 1, dadurch gekennzeichnet, daß man die zu reinigende rohe Glyoxallösung durch die Kammern $K_1$ und die wäßrige Lösung der Base durch die Kammern $K_2$ einer Dialysezelle oder Elektrodialysezelle mit der Sequenz

$$-(-K_2-M_1-K_1-M_1-)_n- \qquad I,$$

in der $M_1$ eine Anionenaustauschermembran und n eine Zahl von 1 bis 800 bedeuten, leitet.

3. Verfahren nach Anspruch 1, dadurch gekennzeichnet, daß man die zu reinigende rohe Glyoxallösung durch die Kammern $K_1$, die wäßrige Base durch die Kammern $K_2$ und einen wäßrigen Elektrolyten durch die Kammern $K_3$ einer Elektrodialysezelle mit der Sequenz

$$-(-M_2-K_2-M_1-K_1-M_1-K_3-)_n- \qquad II,$$

in der $M_2$ eine bipolare Membran oder eine Kationenaustauschmembran und n eine Zahl von 1 bis 800 bedeuten, leitet.

4. Verfahren nach Anspruch 1, dadurch gekennzeichnet, daß man als wäßrige Lösung einer Base die wäßrige Lösung eines Alkali-, Erdalkali- oder Ammoniumcarbonats oder -hydrogencarbonats verwendet.

5. Verfahren nach Anspruch 1, dadurch gekennzeichnet, daß die durch die Kammern der Membranzelle geleiteten Lösungen mit einer Strömungsgeschwindigkeit von 0,001 bis 2 Metern pro Sekunde an der Membranfläche vorbeigeführt werden.

6. Verfahren nach Anspruch 1, dadurch gekennzeichnet, daß die zu reinigende Glyoxallösung einen Glyoxalgehalt von 20 bis 50 Gew.-% und eine Säurezahl von 5 bis 200 aufweist.

## Claims

1. A process for purifying an aqueous glyoxal solution which contains an organic acid by means of a membrane cell comprising two or more compartments separated by ion exchange membranes, which comprises passing the glyoxal solution through at least one of the cell compartments separated from the neighboring compartment(s) by ion exchange membranes and passing the aqueous solution of a base through at least one of the neighboring compartments.

2. A process as claimed in claim 1, wherein the crude glyoxal solution to be purified is passed through compartments $K_1$ and the aqueous solution of the base through compartments $K_2$ of a dialysis or electrodialysis cell comprising the sequence

$$-(-K_2-M_1-K_1-M_1-)_n- \qquad I$$

where $M_1$ is an anion exchange membrane and n is from 1 to 800.

3. A process as claimed in claim 1, wherein the crude glyoxal solution to be purified is passed through compartments $K_1$, the aqueous base through compartments $K_2$ and an aqueous electrolyte through compartments $K_3$ of an electrodialysis cell comprising the sequence

$$-(-M_2-K_2-M_1-K_1-M_1-K_3-)_n- \qquad \text{II}$$

where $M_2$ is a bipolar membrane or a cation exchange membrane and n is from 1 to 800.

4. A process as claimed in claim 1, wherein the aqueous solution of a base is the aqueous solution of an alkali metal, alkaline earth metal or an ammonium carbonate or bicarbonate.

5. A process as claimed in claim 1, wherein the solutions passed through the compartments of the membrane cell flow past the membrane surface at a flow velocity of from 0.001 to 2 meters per second.

6. A process as claimed in claim 1, wherein the glyoxal solution to be purified has a glyoxal content of from 20 to 50% by weight and an acid number of from 5 to 200.

## Revendications

1. Procédé pour l'épuration de solutions aqueuses de glyoxal qui contiennent des acides organiques, à l'aide d'une cellule à membranes se composant d'au moins deux chambres, cellule dans laquelle les chambres sont séparées l'une de l'autre par des membranes échangeuses d'ions, caractérisé en ce qu'on fait passer la solution de glyoxal dans au moins une des chambres de la cellule qui est (sont) séparée(s) de la (des) chambre(s) contiguë(s) par des membranes échangeuses d'anions et en ce qu'on fait passer dans au moins une des chambres contiguës une solution aqueuse d'une base.

2. Procédé selon la revendication 1, caractérisé en ce qu'on fait passer la solution de glyoxal brute à épurer dans les chambres $K_1$ et la solution aqueuse de la base dans les chambres $K_2$ d'une cellule de dialyse ou d'une cellule d'électrodialyse avec la sequence

$$-(-K_2-M_1-K_1-M_1-)_n- \qquad \text{I,}$$

dans laquelle $M_1$ est une membrane échangeuse d'anions et n est un nombre de 1 à 800.

3. Procédé selon la revendication 1, caractérisé en ce qu'on fait passer la solution de glyoxal brute à épurer dans les chambres $K_1$, la base aqueuse dans les chambres $K_2$ et un électrolyte aqueux dans les chambres $K_3$ d'une cellule d'électrodialyse avec la séquence

$$-(-M_2-K_2-M_1-K_1-M_1-K_3-)_n- \qquad \text{II,}$$

dans laquelle $M_2$ représente une membrane bipolaire ou une membrane échangeuse de cations et n est un nombre compris entre 1 et 800.

4. Procédé selon la revendication 1, caractérisé en ce qu'on utilise comme solutions aqueuses d'une base la solution aqueuse d'un carbonate ou d'un hydrogénocarbonate de métal alcalin, de métal alcalino-terreux ou d'ammonium.

5. Procédé selon la revendication 1, caractérisé en ce que les solutions que l'on fait passer dans les chambres de cellule à membranes sont amenées sur la surface de la membrane avec un débit de 0,001 à 2 m/s.

6. Procédé selon la revendication 1, caractérisé en ce que la solution de glyoxal à épurer présente une teneur en glyoxal de 20 à 50% en poids et un indice d'acide de 5 à 200.